# EUROPEAN PATENT APPLICATION

(11) **EP 4 556 003 A1**
(43) Date of publication of application: **21.05.2025**
(21) Application number: 23386117.8
(22) Date of filing: 16.11.2023
(51) Int. Cl.: A61K 31/047, A61K 9/00, A61K 31/133, A61K 47/00, A61P 3/00, A61P 3/04

(54) **ANTI-OBESITY AND ANTI-DIABETIC ACTIVITY OF BROMINATED DITERPENES FROM RED ALGAE OF THE GENUS LAURENCIA AND APPLICATIONS THEREOF**

(71) Applicant: National and Kapodistrian University of Athens, 10679 Athens (GR); Special Account for Research Funds of University of Crete (SARF UoC), 74150 Rethimnon (GR)
(72) Inventor: Ioannou, Efstathia, 15771 Athens (GR); Roussis, Vasileios, 15771 Athens (GR); Harizani, Maria, 15771 Athens (GR); Tsatsanis, Christos, 71003 Heraklio (GR); Paflioti, Eleni, 71003 Heraklio (GR); Panteloglou, Grigorios, 71003 Heraklio (GR); Kampranis, Sotirios C., 71003 Heraklio (GR)
(74) Representative: Vavekis, Konstantinos

(57) **Abstract**

The present invention discloses a method of treatment and/or prevention of obesity using a composition comprising of brominated diterpenes from red algae of the genus *Laurencia* as active ingredients and preferably the diterpene neorogioldiol, isolated from *Laurencia* sp. or chemically synthesized. The said diterpenes suppress pre-adipocyte differentiation to adipocytes, modulate metabolism in 3T3-L1 cells, restrict weight gain and development of glucose intolerance when administered intraperitoneally in vivo, in the mouse model of high fat diet-induced obesity.

## Description

### FIELD OF THE INVENTION

The invention relates to the fields of pharmacy and nutraceutics, namely to the development of a composition for use as a medicament or a dietary supplement; in particular, it concerns a composition comprising of brominated diterpenes from red algae of the genus *Laurencia,* and preferably of the diterpene neorogioldiol, isolated from *Laurencia* sp. or chemically synthesized, as active ingredients for the treatment and/or prevention of obesity.

### BACKGROUND OF THE INVENTION

Obesity is a major disease affecting a high number of individuals worldwide. According to the World Health Organization, in 2016, more than 1.9 billion adults aged 18 years and older were overweight, of these over 650 million adults were obese while, the worldwide prevalence of obesity nearly tripled between 1975 and 2016. Obesity is characterized by excessive fat accumulation and has been associated with premature death and the development of chronic diseases such as type 2 diabetes, hypertension, cardiovascular diseases and some types of cancer [1]. In addition to fat storage, adipose tissue is a major endocrine and metabolic organ secreting adipocytokines, cytokines, growth factors, and hormones that are involved in host immunity, energy homeostasis, systemic insulin sensitivity, and tissue regeneration. The expansion and dysfunction of adipose tissue that take place in obesity contribute to abnormal cytokine and hormone production in adipocytes, resulting in metabolic dysfunction [2].

Expansion of adipose tissue is the result of both the development of mature lipid containing adipocytes from undifferentiated precursors (adipogenesis) and the increase in adipocyte cell size. CCAAT-enhancer-binding protein alpha (CEBPA) and peroxisome proliferator activated receptor gamma (PPARG) are the main regulators of adipogenesis [3, 4]. These two transcription factors regulate the expression of various genes involved in lipogenesis, lipolysis and insulin sensitivity, such as the leptin gene [5], the adiponectin gene [6] and the glucose transporter type 4 (*Slc2a4*) gene [6]. Multiple factors exist that are capable of inhibiting adipogenic differentiation in different cell lines. An example is the preadipocyte factor-1 (DLK1), a transmembrane protein highly expressed in preadipocytes. It prevents lipid accumulation and expression of adipocyte transcription factors, such as PPARG and C/EBPA, and other late adipocyte markers, such as FABP4 [6].

There are several models to study adipogenesis in vitro and in vivo. The murine 3T3-L1 preadipocyte cell line is widely used to study adipocyte differentiation because when differentiated, cells develop a homogenous population of mature adipocytes that are morphologically and biochemically similar to adipocytes in situ [7]. Moreover, it has been shown that non-differentiated 3T3-L1 cells prefer to use oxidative phosphorylation to meet ATP demands and they retain this preference when they differentiate to adipocytes, despite having a large unused glycolytic capacity [8]. Glycolytic capacity is downregulated during adipogenesis programming, since non-differentiated 3T3-L1 cells generate 33% of their ATP by glycolysis, whereas adipocytes generate only 19% of their ATP through glycolysis [8].

Current therapies for weight loss target the orexigenic mechanism, absorption of nutrients at the gastrointestinal tract or mechanisms of insulin signaling and secretion, and to a lesser extent adipocyte differentiation and metabolism. Most chemical compounds used for weight loss have important adverse effects. Therefore, natural products may hold the key for the development of additional drugs or food supplements with fewer or no adverse effects to prevent or ameliorate obesity [9].

### DESCRIPTION OF THE INVENTION

The present invention discloses a method of treatment and/or prevention of obesity using a composition comprising of brominated diterpenes from the red algae of the genus *Laurencia* as active ingredients, and more specifically comprises any of the diterpenes with the chemical structure 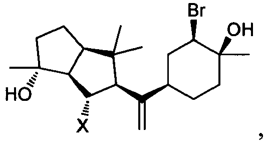 where X = Br, Cl, I, NH₂, and preferably of the diterpene neorogioldiol **(1),** or any combination of them, isolated from *Laurencia* sp. or chemically synthesized. Diterpenes from red algae of the genus *Laurencia* have been isolated and studied for their anti-inflammatory features. Neorogioltriol **(2),** a closely related analogue, exhibits potent analgesic and anti-inflammatory properties in vivo [10, 11], while reducing NFKB1 and COX2 levels in an in vitro model [11]. Two additional related terpenes have been identified from *Laurencia* species, namely neorogioldiol **(1)** and *O*^{11,15}-cyclo-14-bromo-14,15-dihydrorogiol-3,11-diol, which also possess potent anti-inflammatory activity [12]. Although the anti-adipogenic properties of diterpenes and other terpenoids from other plant species have been explored [13, 14], no such studies have been conducted for terpenoids from *Laurencia.* Consequently, the aim of this invention is the use of the above-mentioned diterpenes (as isolated from *Laurencia* or synthetically produced) on the modulation of adipogenesis and cell metabolism in 3T3-L1 cells and in vivo utilizing the high fat-induced obesity animal model. The chemical structures of neorogioldiol **(1)** and neorogioltriol **(2)** isolated from *Laurencia* sp. are as follows:

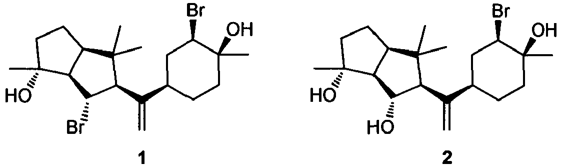

In one embodiment of the invention, the method of prevention and/or treatment of obesity in a mammal (subject) comprises administering to said mammal a pharmaceutical composition comprising a compound (3) of a chemical structure 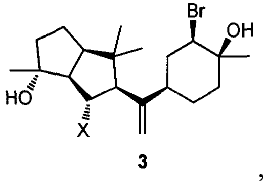 where X = Cl, Br, I, NH₂ and more preferably X=Br (neorogioldiol, **1).** In a further embodiment neorogioldiol **(1)** is extracted from species of the genus *Laurencia.* In another embodiment the compound with the structure **(3)** is synthetically produced.

A further aspect of the invention is the use of a pharmaceutical composition comprising a compound with the structure **(3)** where X = Cl, Br, I, NH₂ and more preferably neorogioldiol **(1)** for its anti-adipogenic effect since they are significantly reducing lipid accumulation resulting in the prevention or/and treatment of a subject for overweight or obesity. More specifically, neorogioldiol **(1)** restricts the increase in glucose transporter 4 (*Slc2a4*)*,* leptin (*Lep*) and adiponectin (*Adipoq*) levels that takes place in mature adipocytes.

In a further embodiment, the invention discloses the use of a pharmaceutical composition comprising compound **3** where X = Cl, Br, I, NH₂ and more preferably neorogioldiol **(1)** as a medicament for inhibiting the differentiation of pre-adipocytes into adipocytes.

In another embodiment, the invention discloses the use of a pharmaceutical composition comprising compound **3** where X = Cl, Br, I, NH₂ and more preferably neorogioldiol **(1)** for exhausting the glycolytic capacity of cells, which affects their adipogenic ability and therefore treats obesity.

In another embodiment the pharmaceutical composition for use in the treatment of obesity comprises of the ethanol extract of *Laurencia* sp. since it contains neorogioldiol **(1)** and other of the said diterpenes.

Another aspect of the invention is that the pharmaceutical composition further comprising a delivery vehicle which enhances cellular uptake. In one embodiment the delivery vehicle is polyethylene glycol and in a preferred embodiment Polyethylene Glycol-400 (Carbowax^{™}-400), well known to the literature to be an effective vehicle for lipid-soluble compounds with no toxicity.

Another aspect of the invention is the administration to a subject (mammal) of the said pharmaceutical compositions. In one embodiment, the diterpene / diterpenes are administered in the form of an injection wherein the compound is dissolved in polyethylene glycol or in any other suitable carrier known in the prior art. In another embodiment, the said diterpene / diterpenes is administered orally in a formulation or composition, such as a dietary supplement, a nutraceutical formulation or a pharmaceutical formulation. Oral administration includes solid formulations, such as tablets, capsules containing particulates, solid solutions, liposomes, or liquid formulations, such as suspensions, solutions or syrups.

### DESCRIPTION OF THE FIGURES

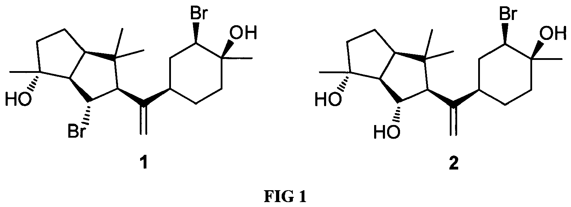

**Fig. 1****.** Structures of neorogioldiol **(1)** and neorogioltriol **(2).**

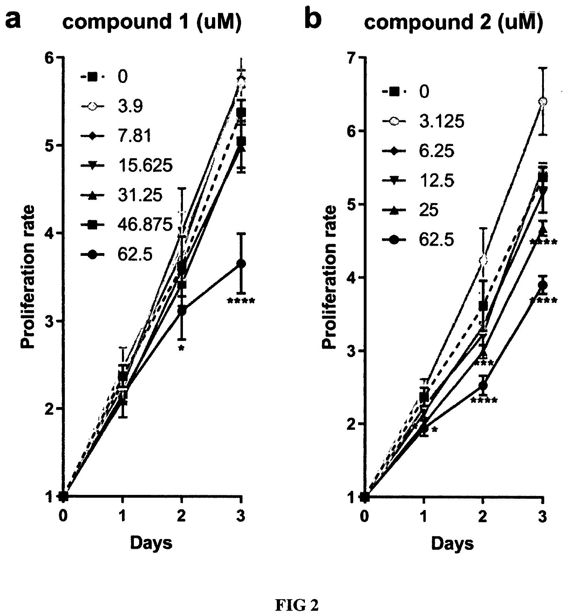

**Fig. 2****.** Evaluation of the effect of neorogioldiol **(1,** n=4) and neorogioltriol **(2,** n=4) on the proliferation of 3T3-L1 cells. Cell proliferation was evaluated using the MTT [3-(4,5-dimethylthiazol-2-yl)-2,5-diphenyltetrazolium bromide] assay and compared to those of cells treated with carbowax 400 0.1% (*v*/*v*). *n* = 5 sample replicates per group. The *p* values were obtained by performing a two-way ANOVA with Dunnett's post hoc test and data are shown as mean ± SD (***p<0.001, ****p<0.0001).

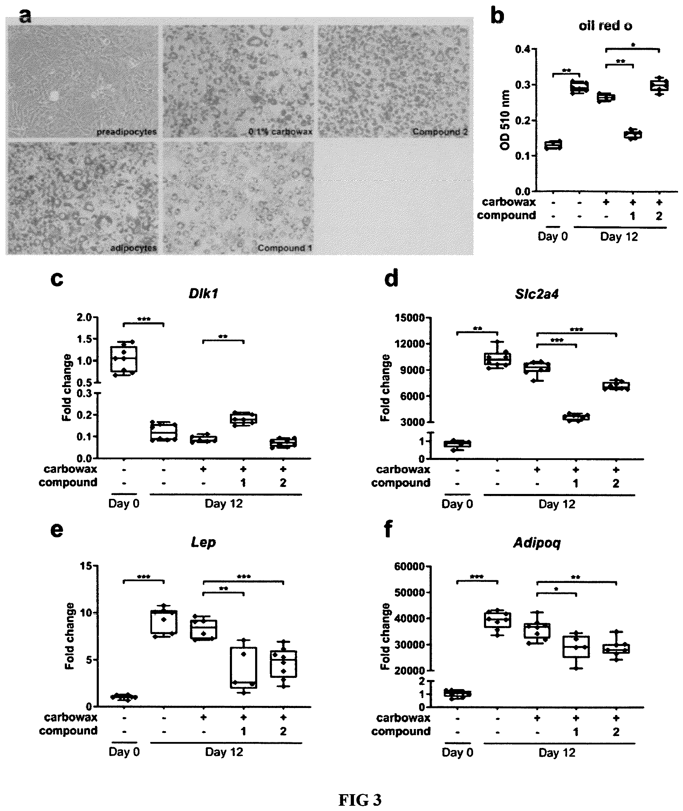

**Fig. 3****.** Effects of neorogioldiol **(1)** and neorogioltriol **(2)** on intracellular lipid accumulation (a-b) and adipocyte gene expression (c-f) during adipocyte differentiation. 3T3L1 preadipocytes were induced to differentiate in the presence of the two compounds. **a**, Representative cell images after oil red o lipid staining at 10 × magnification, **b,** Quantification of oil red o staining. **c-f**, Relative mRNA expression of *Dlkl* (c), *Slc2a4* (d), *Lep* (e), *Adipoq* (f)) in 3T3-L1 adipocytes. *Ppib* was used as internal control gene. Results are representative of two independent experiments (n = 8 sample replicates per group per experiment). The *p* values were obtained by performing a non-parametric Mann-Whitney test and data are shown as mean ± SD (*p<0.05, ***p*<0.01, ***p<0.001).

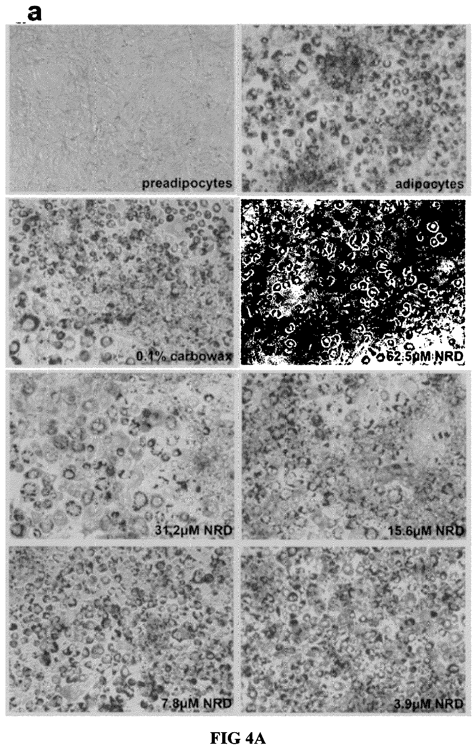

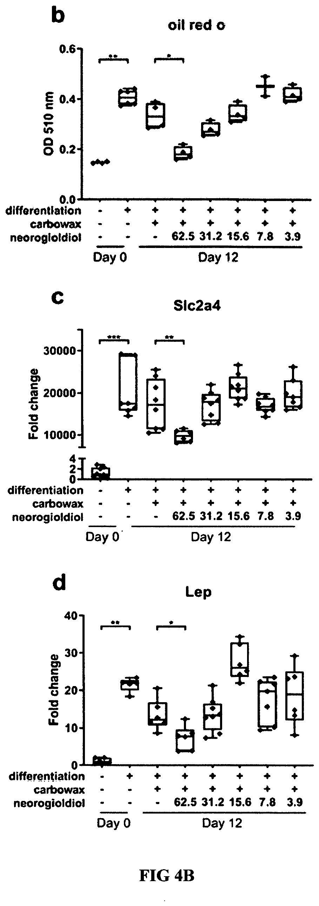

**Fig. 4****.** Dose-dependent effect of neorogioldiol **(1)** on 3T3-L1 preadipocyte differentiation. 3T3L1 preadipocytes were induced to differentiate in the presence of serial neorogioldiol concentrations, 62.5, 31.25, 15.625, 7.81 and 3.9 *µ*M. **a,** Representative cell images after oil red o lipid staining at 10 × magnification, **b,** Quantification of oil red o staining. **c-d**, Relative mRNA expression of *Slc2a4* (c) and *Lep* (d) in 3T3-L1 adipocytes. *Ppib* was used as internal control gene. *n* = 8 sample replicates per group. The *p* values were generated by performing a non-parametric Mann-Whitney test and data are shown as mean ± SD (*p<0.05, **p<0.01, ***p<0.001).

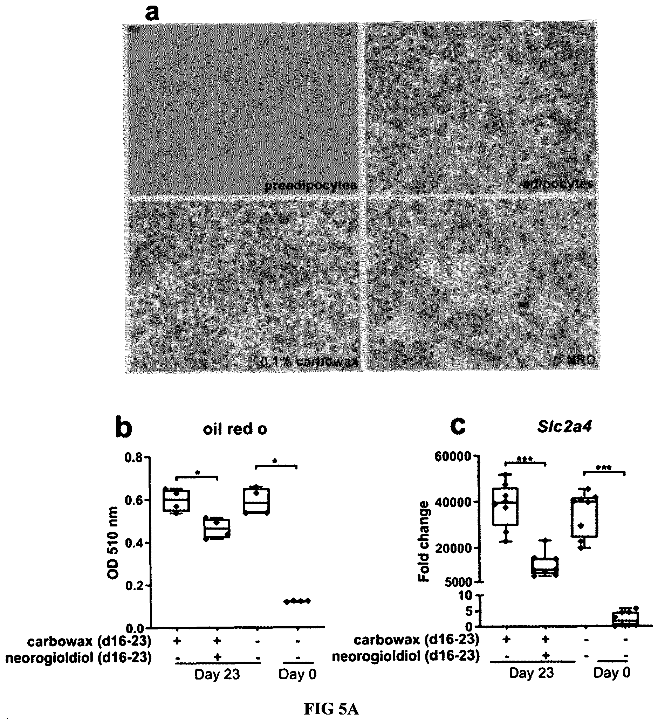

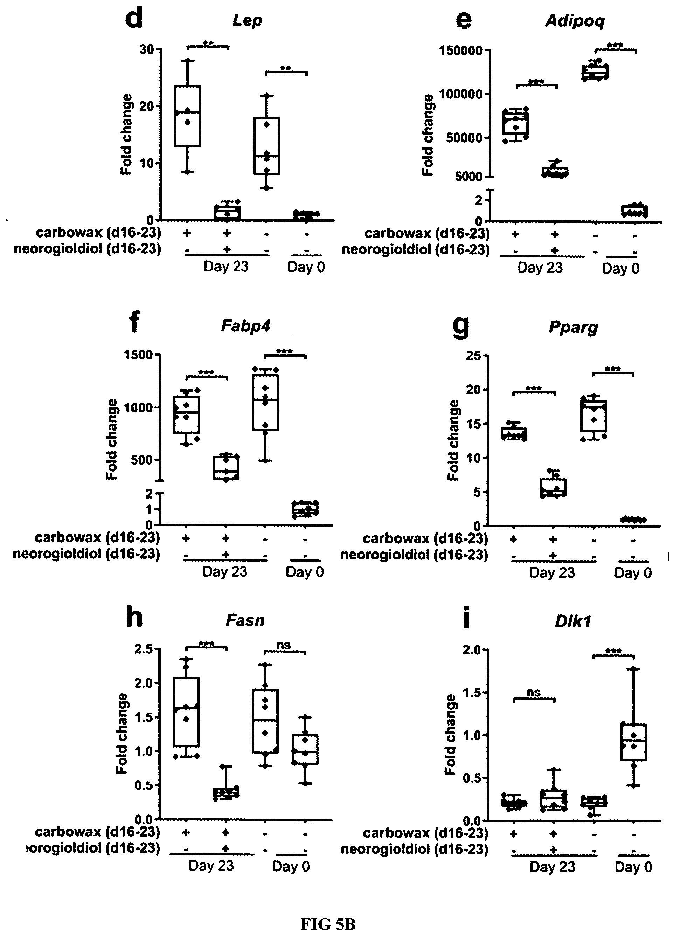

**Fig. 5****.** Effects of neorogioldiol (**1**, 62.5*µ*M) on adipocyte lipid accumulation and gene expression. 3T3L1 preadipocytes were induced to differentiate for 15 days and then neorogioldiol was added to differentiation medium for 8 more days. **a**, Representative cell images after oil red o lipid staining at 10 × magnification. **b,** Quantification of oil red o staining. **c-i**, Relative mRNA expression of *Slc2a4* (c), *Lep* (d), *Adipoq* (e), *Fabp4* (d), *Pparg* (e), *Fasn* (f) and *Dlkl* (g) in 3T3-L1 adipocytes. *Ppib* was used as internal control gene. *n* = 8 sample replicates per group. The *p* values were generated by performing a non-parametric Mann-Whitney test and data are shown as mean ± SD (*p<0.05, **p<0.01, ***p<0.001).

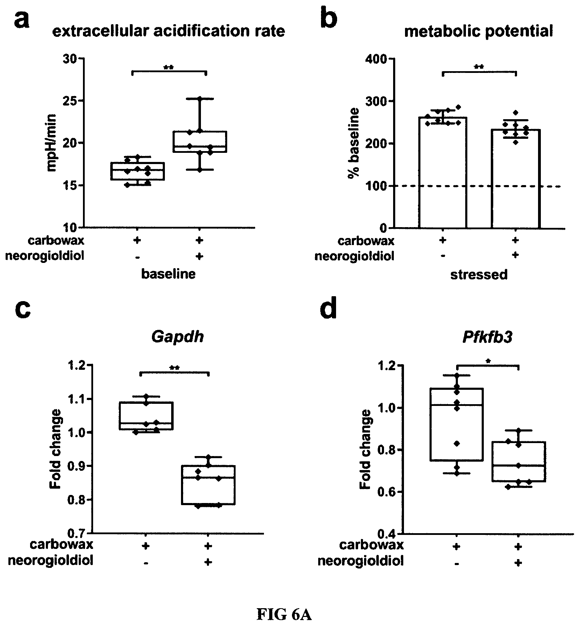

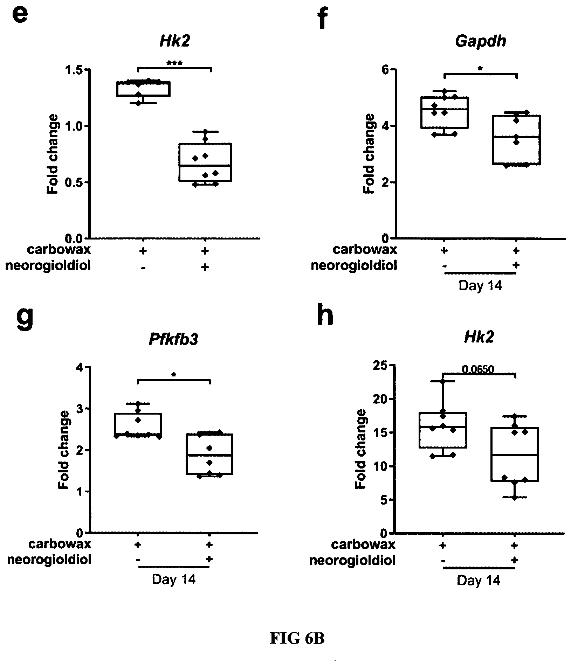

**Fig 6****.** Neorogioldiol **(1)** increased basal glycolysis on preadipocytes but decreased the overall metabolic potential of preadipocytes and adipocytes. 3T3L1 preadipocytes or adipocytes were treated with neorogioldiol for 24 h. **a,** Baseline extracellular acidification rate of preadipocytes after being treated with neorogioldiol for 24 h, **b,** Metabolic potential of preadipocytes expressed as the ratio of extracellular acidification rate under stress to basal extracellular acidification rate. **c-e**, expression of glycolytic enzymes on preadipocytes, after 24 h treatment with neorogioldiol. **f-h**, expression of glycolytic enzymes on adipocytes, after 24 h treatment with neorogioldiol. Results are representative of two independent experiments (*n* = 8 sample replicates per group per experiment). The *p* values were generated by performing a non-parametric Mann-Whitney test and data are shown as mean SD (*p<0.05, ***p*<0.01, ***p<0.001).

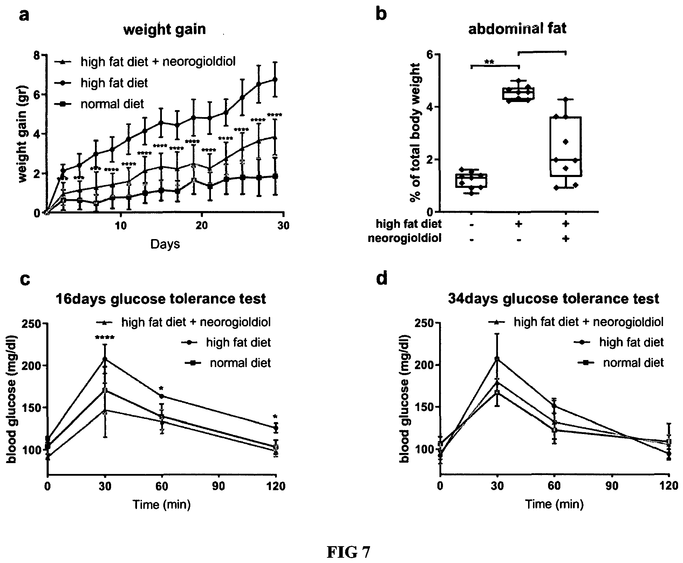

**Fig. 7****.** Neorogioldiol **(1)** limits obesity development in an in vivo high fat diet-induced obesity mouse model. Mice fed a high-fat diet for 35 days with simultaneous administration of neorogioldiol. **a**, Mice weight gain during the 30 days of the experiment. **b**, Abdominal fat weight expressed as a percentage of total body weight. **c,d**, Glucose tolerance test at day 16 (c) and day 34 (d). Results are from two independent experiments with *n* = 9 mice in normal diet group, *n* = 8 mice in high fat diet group and *n* = 9 mice in high fat diet + neorogioldiol group. For the weight gain and the GTTs graphs, *p* values were generated by performing a two-way ANOVA with Dunnett's post hoc test and for the abdominal fat graph by performing a one-way ANOVA with Tukey's post hoc test and data are shown as mean ± SD (*p<0.05, ***p<0.001, ****p<0.0001).

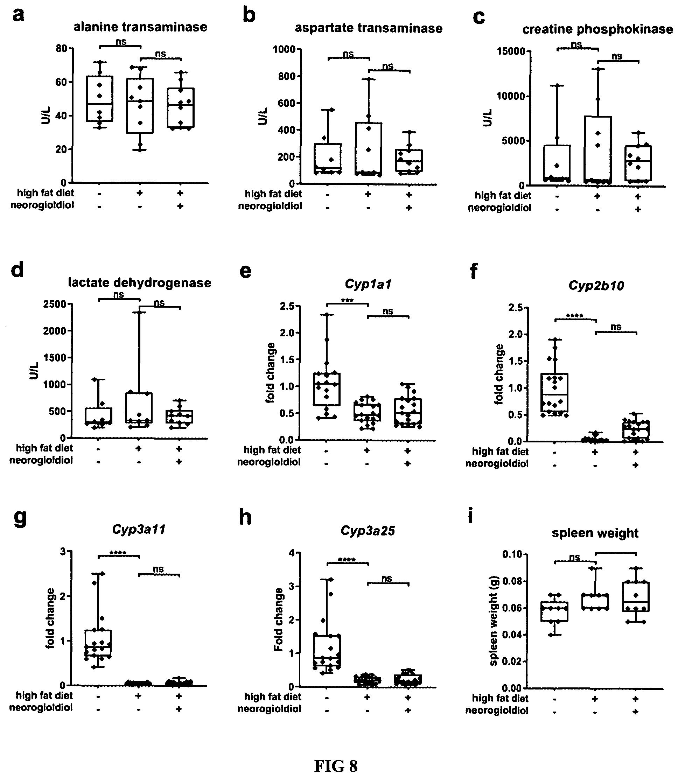

**Fig. 8****.** Neorogioldiol **(1)** did not exhibit any toxicity in vivo. Mice were fed a high-fat diet for 35 days with simultaneous administration of neorogioldiol. **a-d**, Concentration of toxicity biomarkers in mice serum, alanine transaminase (a), aspartate transaminase (b), creatine phosphokinase (c) and lactate dehydrogenase (d). **e-h**, Relative mRNA expression of *Cyp1a1* (e), *Cyp2b10* (f), *Cyp3a11* (g) and *Cyp3a25* (h) in liver RNA extracts. *Actb* was used as internal control gene. i, Mice spleen weight at the end of the experiment. Results are from two independent experiments with *n* = 9 mice in normal diet group, *n* = 8 mice in high fat diet group and *n* = 9 mice in high fat diet + neorogioldiol group. p values were generated by performing a one-way ANOVA with Holm-Sidak's post hoc test and data are shown as mean ± SD (****indicates *p*<0.0001).

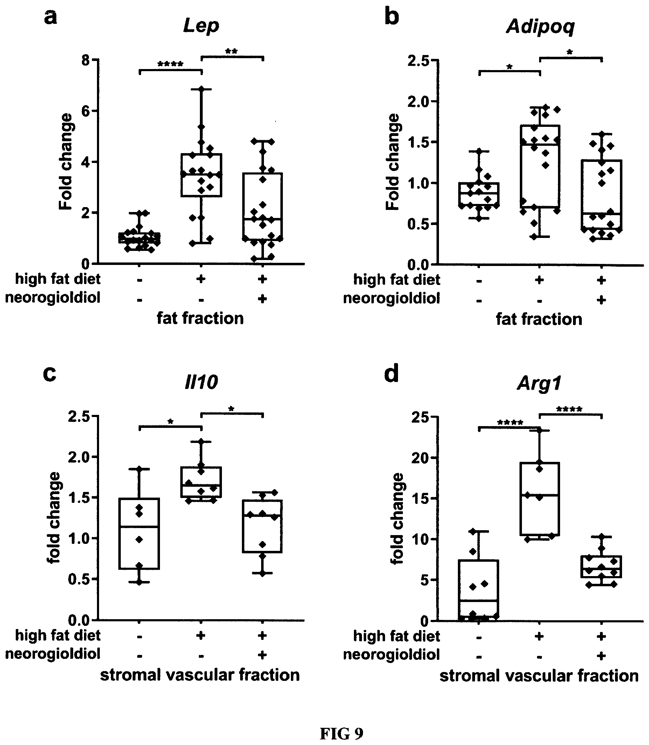

**Fig. 9****.** Effect of neorogioldiol **(1)** on gene expression in abdominal fat. Mice were fed a high-fat diet for 35 days with simultaneous administration of neorogioldiol. **a,b**, Relative mRNA expression of *Lep* (a) and *Adipoq* (b) in the fat fraction of abdominal fat. **c,d**, Relative mRNA expression of *Il10* (c) and *Arg1* (d) in the stromal vascular fraction of abdominal fat. *Ppib* was used as internal control gene in the fat fraction and *Rps9* in the stromal vascular fraction. a,b, Results are from two independent experiments with *n* = 9 mice in normal diet group, *n* = 8 mice in high fat diet group and *n* = 9 mice in high fat diet + neorogioldiol group. c,d, Results are from one experiment with *n* = 4 mice in normal diet group, *n* = 4 mice in high fat diet group and *n* = 5 mice in high fat diet + neorogioldiol group. *p* values were generated by performing a one-way ANOVA with Tukey's post hoc test and data are shown as mean ± SD (*p<0.05, **p<0.01, ****p<0.0001).

### DETAILED DESCRIPTION AND EXAMPLES

### Example 1: Materials and methods for Examples 2-5

### Isolation and identification of metabolites 1 and 2

The fresh algal biomass, collected at Vatsa bay in Kefalonia island, Greece, at a depth of 0.5 to 2 m, was exhaustively extracted with mixtures of CH₂Cl₂/MeOH at room temperature. Evaporation of the solvents under vacuum afforded a dark green oily residue (40 g) that was subjected to vacuum column chromatography on silica gel, using cyclohexane with increasing amounts of EtOAc, which is followed by EtOAc with increasing amounts of MeOH as the mobile phase, to yield 15 fractions (1-15). Fractions eluting with 50-60% EtOAc in cyclohexane were combined (4.8 g) and fractionated by gravity column chromatography on silica gel, using cyclohexane with increasing amounts of EtOAc as the mobile phase, to afford a fraction (15% EtOAc in cyclohexane) that was further purified by normal-phase HPLC, using cyclohexane/Me₂CO (85:15) as eluent, to yield **1 (1** g). Fractions eluting with 100% EtOAc were combined (4 g) and fractionated by gravity column chromatography on silica gel, using CH₂Cl₂ with increasing amounts of Me₂CO, followed by Me₂CO with increasing amounts of MeOH as the mobile phase, to afford a fraction (80% Me₂CO in CH₂Cl₂, 200 mg) that was purified by normal-phase HPLC, using cyclohexane/EtOAc (40:60) as eluent, to yield **2** (19 mg). The isolated compounds were identified as neorogioldiol **(1)** and neorogioltriol **(2)** by comparison of their spectroscopic and physical characteristics with those reported in the literature [12].

### Cells culture and differentiation

Mouse 3T3-L1 preadipocytes were maintained in proliferation medium containing DMEM supplemented with 10% (v/v) heat inactivated NBCS and 1% (v/v) penicillin-streptomycin at 37 °C with 5% CO₂, humidified atmosphere. Cells were seeded in T-75 flasks at a density of 25×10⁴ cells/flask. Cultures never became completely confluent according to the manufacturer's instructions. They were passaged using trypsin-EDTA solution once they reached 70-80% confluency. For differentiation, 3T3-L1 cells were seeded and after 24 h (day 1), cell differentiation was induced by changing the medium to high glucose DMEM containing 10% (v/v) heat inactivated FBS, 1% penicillin-streptomycin, 10 *µ*g/mL insulin, 0.25 *µ*M Dexamethasone (GAP), 0.5 mM IBMX (=differentiation medium I). On day 3, the medium was changed to high glucose DMEM containing 10% FBS, 1% penicillin- streptomycin, and 10 *µ*g/mL insulin (=differentiation medium II). Thereafter, medium was changed every other day switching between differentiation medium I and differentiation medium II until day 12 or 23, when adipocytes were harvested. Control group preadipocytes were seeded at a higher density and harvested after two days. Test compounds were administered either at the initiation of differentiation, or after 15 days of differentiation and with every medium change. To determine the effect on preadipocyte differentiation, cells were seeded and after 24 h they were treated with neorogioldiol. Neorogioldiol was maintained in the culture medium throughout cell differentiation period. To determine the effect of neorogioldiol on mature adipocytes, cells were seeded and induced to differentiate for 14 days, then on day 15 neorogioldiol was added to the differentiation medium for 24 h before cells were harvested. Each compound used was diluted in a 90% carbowax^{™} 400, 10% (v/v) absolute ethanol solution serving as a control solvent. The final concentration in culture was 0.09% (v/v) carbowax and 0.01% (v/v) ethanol. Compounds were used at the following concentrations: neorogioldiol **(1)** 62.5*µ*M and neorogioltriol (2) 25*µ*M.

### Oil red o staining

Oil red o staining was applied to measure lipid accumulation intracellularly. Briefly, after 12 or 23 days of differentiation, the cell culture medium was removed and the 3T3-L1 adipocytes were fixed in 3.7% formaldehyde for 60 min at room temperature. Next, a solution of 0.21% oil red o was filtered using a 0.45 *µ*m filter followed by incubation of the fixed cells for 45 min at room temperature. Excess dye was removed and cells were washed twice with PBS. Fresh PBS was added and the lipid droplets within the differentiated 3T3-L1 cells were visualized and photographed by microscopy. For quantitative analysis, oil red o staining was eluted with 100% isopropanol and the absorbance was measured at 510 nm using the Infinite^{®} 200 PRO microplate plate reader (Tecan, Zurich, Switzerland).

### Cell viability

The cell viability was evaluated using the MTT assay. 3T3-L1 cells were seeded at a density of 1×10³ in 96-well plates and incubated for 24h. Compounds 1 and 2 were added in the wells at various concentrations and then incubated at 37 °C. Cells were treated with compound 1 at concentrations of 62.5, 46.875, 31.25, 15.625, 7.81 and 3.9 *µ*M. Cells were treated with compound 2 at concentrations of 62.5, 25.0, 12.5, 6.25 and 3.125 *µ*M. After 24, 48, and 72 h of treatment, MTT solution was added in the wells at a final concentration of 0.5 mg/mL and incubated for 4 h. Cell culture media with MTT was removed and the formazan crystals were dissolved in 100 *µ*l/well isopropanol/HCl (0.4%) and the absorbance was measured at 600 nm using the Infinite^{®} 200 PRO microplate plate reader (Tecan, Zurich, Switzerland).

### Cell metabolism analysis

Seahorse XF Cell Energy Phenotype Test Kit and Seahorse XF Cell Mito Stress Test Kit were used to measure oxygen consumption rate and extracellular acidification rate on a Seahorse Bioscience XF Analyzer (Agilent Technologies). 3T3-L1 preadipocytes were seeded at 7.5×10³ per well in growth medium in XF 96 Cell Culture Microplate (Agilent Technologies) two days prior to assay. 24 h prior to the assay, medium was changed and compound 1 was added at concentration of 62.5 *µ*M. The day of the assay, cells were washed once and media was replaced with XF Base Medium (pH 7.4) supplemented with 1 mM pyruvate, 2 mM glutamine and 10 mM glucose. The plate was placed in a 37 °C incubator without CO₂ for one hour prior to the assay. Cells were washed a second time right before starting the experiment. After measuring basal oxygen consumption rate and extracellular acidification rate, oligomycin (1.0 *µ*M) and FCCP (1.0 *µ*M) were introduced in real time to measure oxygen consumption rate and extracellular acidification rate under stressed condition. For the baseline extracellular acidification rate the third out of the three baseline measurements was used, while for the stressed condition, the highest measurement after the injections was used. The metabolic potential is calculated as the percentage of stressed extracellular acidification rate to baseline extracellular acidification rate.

### Total RNA isolation, cDNA synthesis and quantitative PCR for cell culture samples

Adipocytes and preadipocytes were harvested using the TRI Reagent^{®}. RNA isolation was carried out according to the manufacturer's instructions. The purified RNA was quantified using the Nanodrop micro-volume spectrophotometer and 500 ng of each sample were used as a template for reverse transcription. cDNA synthesis was performed using the PrimeScriptTM RT reagent Kit (TaKaRa, RR037A) according to manufacturer's instructions. The cDNA produced by the above described procedure was diluted and used as a template for two-step quantitative PCR in a StepOnePlus^{™} Real-Time PCR System (Applied Biosystems^{™}, 4376600) for a maximum of 40 cycles, as follows: start step 95 °C for 3 min, repeat steps: 95 °C for 15 s and 60 °C for 30 s. Amplification was performed using KAPA SYBR^{®} FAST Universal qPCR kit (Kapa Biosystems, KK4618) according to manufacturer's instructions. The primers used are listed in Table 1. Relative quantitative analysis was accomplished calculating fold-changes of gene expression as 2-(ΔΔCt), where ΔCt is (Ct (gene of interest) - Ct (housekeeping gene)). *Ppib* was used as internal control gene in 3T3L1 cells and in the fat fraction of mice abdominal fat, *Rps9* was used as internal control gene in the stromal vascular fraction of mice abdominal fat and *Actb* was used as internal control gene in liver samples.

**Table 1. List of primers used for quantitative real-time PCR analysis.**

| **Primers** | **Forward** | **Reverse** |
|---|---|---|
| *Slc2a4* | GCCCCATTCCCTGGTTCATT | GACCCATAGCATCCGCAACA |
| *Lep* | TTCACACACGCAGTCGGTAT | TCATTGGCTATCTGCAGCAC |
| *Adipoq* | AAAGGAGATGCAGGTCTTCTTG | CTGAGCGATACACATAAGCGG |
| *Dlk1* | AAGTGTGTAACTGCCCCTGG | TGCAAGCCCGAACGTCTATT |
| *Fabp4* | GCTCCTCCTCGAAGGTTTACAA | AATCCCCATTTACGCTGATGAT |
| *Pparg* | GCTGGCCTCCTTGATGAATA | TTGGGCTCCATAAAGTCACC |
| *Fasn* | GACTCGGCTACTGACACGAC | CGAGTTGAGCTGGGTTAGGG |
| *Gapdh* | CTGCACCACCAACTGCTTAG | GGGCCATCCACAGTCTTC |
| *Pfkfb3* | TCACCGACCCTCGTTGCCCA | TGCTTCACAGCCTCACGCCG |
| *Hk2* | GAGAACCGTGGACTGGACAA | GACACGTCACATTTCGGAGC |
| *Il10* | GCGCTGTCATCGATTTCTCCCCTG | GGCCTTGTAGACACCTTGGTCTTGG |
| *Arg1* | ACCTGCTGGGAAGGAAGAAAAGGCC | AAGCCACTGCCGTGTTCACAGTAC |
| *Ppib* | ACAGCAAGTTCCATCGTGTCAT | GAAGAACTGTGAGCCATTGGTG |
| *Actb* | CATTGCTGACAGGATGCAGAAGG | TGCTGGAAGGTGGACAGTGAGG |

### Mice

All procedures were conducted in compliance with protocols approved by the Animal Care Committee of the University of Crete, School of Medicine (Facility number EL91-BIOexp-06), in conjunction with the Veterinary Department of the Region of Crete, Heraklion, Crete, Greece. C57BL/6 adult male mice were maintained in a 12 h day/night cycle and 21-23 °C conditions. Mice were provided with water and either normal fat diet, or high fat diet (60% energy from fat) ad libitum. All mice were weighed every 48 h.

Neorogioldiol was first dissolved in 90% carbowaxTM 400, 10% (v/v) absolute ethanol solution and then diluted 1:10 in water for injection. A dose of 0.25 mg neorogioldiol in a total volume of 200 *µ*l was administered intraperitoneally every 48 h in mice in high fat + neorogioldiol group starting from day 1. 4-5 mice were used per group and each experiment was performed twice.

### Glucose tolerance test

Mice were fasted for 16 h before receiving an intraperitoneal injection of 35% dextrose (100 *µ*l / 35 gr). Blood glucose concentrations were measured in blood collected by venous bleeding from the tail vein immediately before the injections and after 30, 60 and 120 min.

### Adipose tissue fraction separation

Abdominal fat lobes were weighed, washed in PBS, minced and dissolved with collagenase. They were incubated in 1.5mg/ml collagenase in 1% PBS-BSA, for 30 min at 37 °C. Next, homogenate was centrifuged at 800 rpm for 5 min at room temperature. The upper fatty phase, which contains the adipocytes, was collected and resuspended in TRI Reagent^{®} and RNA isolation was carried out according to the manufacturer's instructions. The cell pellet, which is the stromal vascular fraction, was washed once with PBS and resuspended in TRI Reagent^{®} for RNA isolation.

### Determination of biochemical markers

To detect serum levels of alanine transaminase, aspartate transaminase, creatine phosphokinase and lactate dehydrogenase, serum was collected from peripheral blood via cardiac puncture under isoflurane anesthesia. Serum samples were stored at -80 °C. The levels of alanine transaminase, aspartate transaminase, creatine phosphokinase and lactate dehydrogenase were measured by standard enzymatic procedures using an automated biochemical analyzer AU5800 (Beckman).

### Statistical analysis

Statistical analysis was performed using GraphPad Prism version 8.02 for Windows, GraphPad Software, La Jolla California USA, www.graphpad.com.

### Synthetic methods

The total synthesis of neorogioldiol-related diterpenes (Scheme 1) can be accomplished through palladium catalyzed enantioselective vinylation of the β-hydroxy ketone v by the vinyl triflate ix.

Enol triflate ix would be obtained from the corresponding methyl ketone which in turn can be formed from **viii** through a Lemieux-Johnson type oxidation. Intermediate **viii** can be synthesized from limonene oxide [15].

Organocatalytic Michael addition of dimethyl malonate **ii** to 4,4'- dimethyl cyclopentenone i would lead to the optically active cyclopentenone **iii.** Mono decarboxylation of **iii** would afford the corresponding keto ester followed by protection of the carbonyl group as a ketal, reduction of the ester to the corresponding aldehyde and one carbon elongation through a Wittig reaction and deprotection of the ketone would lead to iv. Intramolecular acid catalyzed aldol condensation of **iv** would lead the hydroxy ketone **v.**

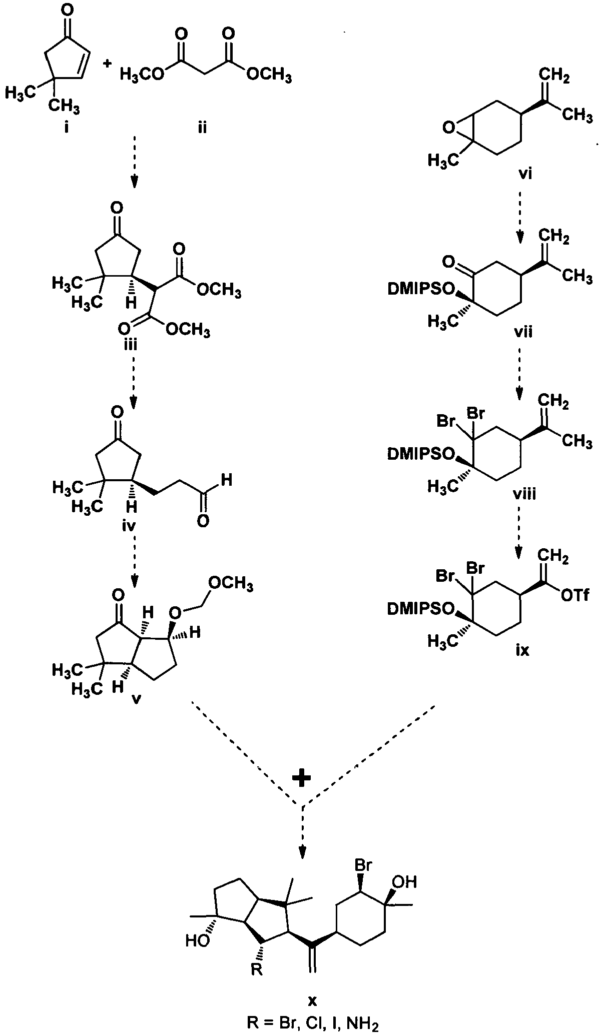

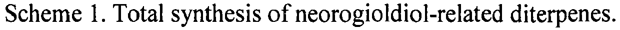

### Example 2: Effects on differentiation of preadipocytes and intracellular lipid accumulation

Initially, the impact of increasing concentrations of **1** and **2** (Figure 1) on cell viability and proliferation during a three-day incubation period was measured using the MTT [3-(4,5-dimethylthiazol-2-yl)-2,5-diphenyltetrazolium bromide] assay. Neorogioldiol **(1)** was cytostatic only at the concentration of 62.5 *µ*M after the first 24 h of treatment (Figure 2a). Neorogioltriol **(2)** exhibited cytostatic effects in concentrations above 25 *µ*M during the incubation period (Figure 2b). Thus, further analyses were performed at concentrations that did not exhibit cytotoxicity, which was 62.5 *µ*M for compound **1** and 25 *µ*M for compound **2.**

Subsequently, the effects of compounds **1** and **2** on adipocyte differentiation were studied. For that purpose, differentiation was induced in the presence of compounds **1** and **2** and after 12 days, lipid accumulation in 3T3-L1 cells was measured. Neorogioldiol **(1)** significantly reduced lipid accumulation, while neorogioltriol **(2)** increased lipid accumulation (Figure 3a,b). The effect of the compounds on mRNA expression of adipocyte differentiation genes was measured. Expression of preadipocyte factor-1 (*Dlkl*)*,* which is dramatically reduced in adipocytes, was diminished in cells differentiated in the presence of compound **1,** but not in the control (Figure 3c). Compound **2** did not change *Dlkl* expression (Figure 3c). Neorodioldiol **(1)** and neorogioltriol **(2)** restricted the increase in glucose transporter 4 (*Slc2a4*)*,* leptin (*Lep*) and adiponectin (*Adipoq*) expression levels, hallmarks of adipocyte differentiation (Figure 3d-f). After establishing the anti-adipogenic effect of neorogioldiol **(1)** on 3T3-L1 adipocytes, differentiation was induced in the presence of serial concentrations of neorogioldiol. Neorogioldiol reduced lipid accumulation (Figure 4a,b) and restricted the increase in *Slc2a4* and *Lep* levels in a dose-dependent manner, at concentrations of 62.5, 31.25, 15.625, 7.81 and 3.9 *µ*M (Figure 4c,d).

### Example 3: Effects of neorogioldiol on adipocyte lipid accumulation and gene expression

To further investigate the effect of neorogioldiol (1) on 3T3-L1 mature adipocytes, adipocyte differentiation was initially induced for 15 days and then neorogioldiol was added to the differentiation medium for 8 more days. Neorogioldiol decreased lipid accumulation (Figure 5) and also decreased the expression of genes that are induced in mature adipocytes highlighting differentiation, such as *Slc2a4, Lep, Adipoq, Fabp4, Pparg* (Figure 5a-e). Neorogioldiol did not affect the expression of *Dlkl* in mature adipocytes (Figure 5g), which probably suggests that 8 days in the presence of neorogioldiol are not enough for cells to restore *Dlkl* expression.

### Example 4: Effects of neorogioldiol on preadipocytes metabolism

To explore the effects of neorogioldiol **(1)** on 3T3-L1 cell metabolism, preadipocytes were treated with 62.5*µ*M neorogioldiol for 24 h. Extracellular acidification rate, which reflects the rate of glycolysis of the cells, was measured using a Seahorse XF Analyzer. Neorogioldiol **(1)** shifted cells to a more glycolytic phenotype, increasing basal levels of extracellular acidification rate (Figure 6a). Following oligomycin and FCCP injections, normally extracellular acidification rate (glycolysis) is increased because mitochondrial respiration is disturbed. Neorogioldiol decreased the metabolic potential of cells (Figure 6b) by exhausting their ability to produce ATP through glycolysis under stress.

To support these findings, the expression of genes involved in the glycolysis pathway was measured. Neorogioldiol downregulated glyceraldehyde-3-phosphate dehydrogenase (*Gapdh*)*,* 6-phosphofructo-2-kinase/fructose- 2,6-biphosphatase 3 (*Pfkfb3*) and hexokinase 2 (Hk2) expression (Figure 6c-e). The decreased expression levels of glycolysis enzymes may be correlated with the lower metabolic potential of cells treated with neorogioldiol.

Next, to explore the effect of neorogioldiol **(1)** on adipocyte metabolism, adipocytes were treated with neorogioldiol (62.5*µ*M) for 24 h and the expression of the same glycolysis genes were measured. Neorogioldiol exerted the same effect on adipocytes as on preadipocytes, decreasing *Gapdh, Pfkfb3* and *Hk2* expression (Figure 6f-h). These results indicate that neorogioldiol exhausts the glycolytic capacity of the cells, which readily affects their adipogenic ability.

### Example 5: Neorogioldiol limits obesity development in an in vivo high fat diet-induced obesity mouse model

To study neorogioldiol **(1)** in vivo, a high fat diet-induced obesity mouse model with simultaneous administration of neorogioldiol was employed. Mice treated with intraperitoneal injection of neorogioldiol (0.25 mg/mouse, every 48 h for 35 days) exhibited suppressed weight gain throughout the 35 days of the experiment (Figure 7a), resulting in reduced abdominal fat mass (Figure 7b) as compared to mice fed high fat diet without neorogioldiol administration, demonstrating that neorogioldiol **(1)** limits the development of obesity in vivo.

In order to evaluate whether neorogioldiol administration causes toxicity in vivo, alanine transaminase and aspartate transaminase, which are known biomarkers of hepatotoxicity, were measured in the serum of animals treated with neorogioldiol by intraperitoneal injection (0.25 mg/mouse, every 48 h for 35 days) [16]. In addition, creatine phosphokinase and lactate dehydrogenase, which are biomarkers for tissue damage, including muscle damage (rhabdomyolysis), were measured in the serum of mice [17, 18]. No difference in these biomarkers was observed between mice treated with neorogioldiol and the control group (Figure 8a-d). To examine liver toxicity, the expression of cytochrome P450 genes (*Cyp1a1, Cyp2b10, Cyp3a11, Cyp3a25),* was measured in liver RNA extracts. The hepatic cytochrome P450 families are involved in drug metabolism and pharmacokinetics, as well as the pathogenesis of liver diseases [19] and it has been shown that their mRNA levels are reduced in high fat diet treatment [20]. Figure 8e-h, demonstrates that indeed their expression levels are decreased in mice fed a high-fat diet, but there is no difference with the neorogioldiol group. Spleen weight is considered to be an indicator of inflammatory responses, when spleen weight is increased, or immunotoxicity, when spleen weight is reduced [21]. Spleen weight was similar between all groups (8i). Overall, neorogioldiol (1) did not exhibit any toxicity in vivo. To evaluate the development of glucose intolerance, glucose tolerance test (GTT) was performed at day 16 and day 34, one day before protocol termination. The results showed that after 16 days, mice administered with neorogioldiol (0.25 mg/mouse, every 48 h for 35 days) had not developed glucose intolerance (Figure 7c), whereas on day 34, as they continued gaining weight, they became glucose intolerant (Figure 7d). These results suggest that, although neorogioldiol initially prevents obesity-related glucose intolerance, by preventing weight gain, it cannot prohibit development of glucose intolerance at the late stages of obesity. To further investigate the effect of neorogioldiol on adipose tissue homeostasis in vivo, the expression of adipocyte differentiation and inflammatory gene expression was determined in adipose tissue fat and the stromal vascular fraction, respectively. After isolating abdominal fat, fat and stromal vascular fraction were separated and RNA was extracted. *Lep* and *Adipoq* expression was upregulated in the fat fraction in high fat diet-fed mice compared to normal diet-fed mice, while high fat diet-induced obese mice administered with neorogioldiol had lower levels of *Lep* and *Adipoq* as compared to untreated obese mice (Figure 9a,b). Additionally, *Il10* and *Arg1* expression was increased in the stromal vascular fraction from high fat diet-induced obese mice as compared to control mice fed with normal diet, while it was downregulated in high fat diet-fed mice administered with neorogioldiol, as compared to high fat diet mice (Figure 9c,d). High fat diet-induced obesity mouse models represent different stages of obesity where they develop glucose intolerance within two weeks and gradual increase of adipose tissue mass reaching plateau at 35 days [22]. Thus, after 4 weeks obesity is at the early stages resembling a pre-diabetes stage, having an early-obesity metabolically impaired phenotype. Taking that into account, *Lep* and *Adipoq* increased expression levels can be attributed to the expansion of adipose tissue and the development of new mature adipocytes from undifferentiated precursors. Mice administered with neorogioldiol had lower levels of *Lep* and *Adipoq* in the fat fraction of abdominal fat compared to mice only fed with high fat diet (Figure 9a,b), which suggests that they are at an earlier stage of obesity, consistently with the lower weight gain and decreased abdominal fat weight (Figure 7a,b). Lumeng et al. [23] described an early stage of mild obesity, in which while adipocytes undergo hypertrophy and release chemokines that induce recruitment of M1-polarized macrophages, M2-polarized resident adipose tissue macrophages are expanding as a negative feedback mechanism to protect adipocytes from these inflammatory factors and block M1 polarization. Consequently, the increase in *Il10* and *Arg1* expression levels in the stromal vascular fraction of high fat diet-fed mice suggests that resident M2 macrophages are expanding to maintain a healthy adipose tissue. Neorogioldiol, by delaying obesity development, maintains *Il10* and *Arg1* levels similar to the control levels (Figure 9c,d). Overall, mice administered with neorogioldiol gained less weight and maintained a closer to normal gene expression in abdominal fat.

### References

1. Friedman, J. M. Obesity in the new millennium. Nature, 2000, 404, 632.
2. Coelho, M., Oliveira, T. & Fernandes, R. Biochemistry of adipose tissue: an endocrine organ. Archives of medical science: AMS, 2013, 9, 191.
3. Rosen, E. D. & MacDougald, O. A. Adipocyte differentiation from the inside out. Nature Reviews Molecular Cell Biology 2006, 7, 885-896.
4. Farmer, S. R. Transcriptional control of adipocyte formation. Cell Metabolism, 2006, 4, 263-273.
5. Hwang, C.-S., Loftus, T. M., Mandrup, S. & Lane, M. D. Adipocyte differentiation and leptin expression. Annual Review of Cell and sevelopmental Biology, 1997, 13, 231-259.
6. Moseti, D., Regassa, A. & Kim, W.-K. Molecular regulation of adipogenesis and potential anti-adipogenic bioactive molecules. International Journal of Molecular Sciences, 2016, 17, 124.
7. Sarjeant, K. & Stephens, J. M. Adipogenesis. Cold Spring Harbor Perspectives in Biology, 2012, 4, a008417.
8. Guntur, A. R. et al. Osteoblast-like MC3T3-E1 cells prefer glycolysis for ATP production but adipocyte-like 3T3-L1 cells prefer oxidative phosphorylation. Journal of Bone and Mineral Research, 2018, 33, 1052-1065.
9. Fu, C., Jiang, Y., Guo, J. & Su, Z. Natural products with anti-obesity effects and different mechanisms of action. Journal of Agricultural and Food Chemistry, 2016, 64, 9571-9585.
10. Chatter, R. et al. Neorogioltriol: A brominated diterpene with analgesic activity from Laurencia glandulifera. Phytochemistry Letters, 2009, 2, 25-28.
11. Chatter, R. et al. In vivo and in vitro anti-inflammatory activity of neorogioltriol, a new diterpene extracted from the red algae Laurencia glandulifera. Marine Drugs, 2011, 9, 1293-1306.
12. Daskalaki, M. G. et al. Neorogioltriol and related diterpenes from the red alga Laurencia inhibit inflammatory bowel disease in mice by suppressing M1 and promoting M2-like macrophage responses. Marine Drugs, 2019, 17, 97.
13. Kim, J. S., Lee, S. G., Kang, Y. J., Kwon, T. K. & Nam, J.-O. Kahweol inhibits adipogenesis of 3T3-L1 adipocytes through downregulation of PPARγ. Natural Product Research, 2018, 32, 1216-1219.
14. Bu, S., Yuan, C. Y., Xue, Q., Chen, Y. & Cao, F. Bilobalide suppresses adipogenesis in 3T3-L1 adipocytes via the AMPK signaling pathway. Molecules, 2019, 24, 3503.
15. Blair, M., Forsyth, C.M., Tuck, K.L. Towards the synthesis of prevezol C: total enantioselective synthesis of (-)-2-epi-prevezol C. Tetrahedron Lett., 2010, 51, 4808-4811.
16. Ozer, J., Ratner, M., Shaw, M., Bailey, W. & Schomaker, S. The current state of serum biomarkers of hepatotoxicity. Toxicology, 2008, 245, 194-205.
17. Beigvand, H. H., Heidari, K., Hashemi, B. & Saberinia, A. The value of lactate dehydrogenase in predicting rhabdomyolysis-induced acute renal failure; a narrative review. Archives of Academic Emergency Medicine 2021, 9, e24.
18. Bagley, W., Yang, H. & Shah, K. Rhabdomyolysis. Internal and Emergency Medicine, 2007, 2, 210-218.
19. Villeneuve, J.-P. & Pichette, V. Cytochrome P450 and liver diseases. Current Drug Metabolism, 2004, 5, 273-282.
20. He, Y. et al. High fat diet significantly changed the global gene expression profile involved in hepatic drug metabolism and pharmacokinetic system in mice. Nutrition & Metabolism 2020, 17, 1-15.
21. Investigators 12, T. I. G. Report of validation study of assessment of direct immunotoxicity in the rat. Toxicology, 1998, 125, 183-201.
22. Li, J., Wu, H., Liu, Y. & Yang, L. High fat diet induced obesity model using four strains of mice: Kunming, C57BL/6, BALB/c and ICR. Experimental Animals, 2020, 69, 326-335.
23. Lumeng, C. N., Bodzin, J. L., Saltiel, A. R., et al. Obesity induces a phenotypic switch in adipose tissue macrophage polarization. The Journal of Clinical Investigation, 2007, 117, 175-184.

## Claims

1. A pharmaceutical composition comprising a compound with the chemical structure 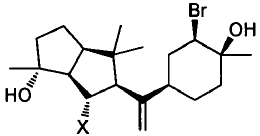 where X = Cl, Br, I, NH₂ and more preferably where X = Br, neorogioldiol, for use in the prevention/treatment of obesity in a subject.

2. A pharmaceutical composition for use in the prevention/treatment of obesity in a mammal according to claim 1 wherein the compound is neorogioldiol extracted from *Laurencia* sp.

3. A pharmaceutical composition for use in the prevention/treatment of obesity in a mammal according to claim 1 wherein the compound is synthetically produced.

4. A pharmaceutical composition comprising a compound with the chemical structure 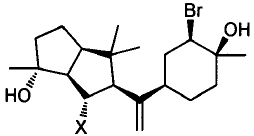 where X = Cl, Br, I, NH₂ and more preferably neorogioldiol for use in the restriction of the increase in glucose transporter (*Slc2a4*)*,* leptin (*Lep*)*,* and adiponectin (*Adipoq*) levels that takes place in mature adipocytes in a mammal.

5. A pharmaceutical composition comprising a compound with the chemical structure 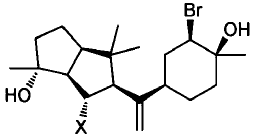 where X = Cl, Br, I, NH₂ and more preferably neorogioldiol for inhibiting the differentiation of pre-adipocytes into adipocytes in a mammal.

6. A pharmaceutical composition comprising a compound with the chemical structure 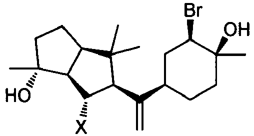 where X = Cl, Br, I, NH₂ and more preferably neorogioldiol for exhausting the glycolytic capacity of pre-adipocytes, which affects their ability to differentiate into mature adipocytes, and suppression of the glycolytic mechanism of mature adipocytes, in a mammal.

7. A pharmaceutical composition for use in the prevention/treatment of obesity according to any of the claims 1 to 6, which comprises an ethanol extract of red algae of the genus *Laurencia.*

8. A pharmaceutical composition for use in the prevention/treatment of obesity according to any of the previous claims which further comprises a delivery vehicle that enhances cellular uptake, specifically polyethylene glycol and even more specifically Polyethylene Glycol-400.

9. A method of prevention or treatment of obesity comprising the step of administering to the subject a pharmaceutically effective amount of the pharmaceutical composition of any of the claims 1 to 8.

10. A method of prevention or treatment of obesity, according to claim 9, wherein the administration of the said pharmaceutical composition of any of the claims 1 to 8, is administered in the form of an injection of the said diterpene dissolved in polyethylene glycol or in any other suitable carrier.

11. A method of prevention or treatment of obesity, according to claim 9, wherein the administration of the said pharmaceutical composition of any of the claims 1 to 8, is administered orally in a formulation or composition, such as a dietary supplement, a nutraceutical formulation or a pharmaceutical formulation.

12. A method of prevention or treatment of obesity, according to claim 11, wherein the administration of the said pharmaceutical composition of any of the claims 1 to 8, is administered orally in the form of a solid formulation and more preferably as tablets, or as capsules containing particulates, or as solid solutions, or as liposomes.

13. A method of prevention or treatment of obesity, according to claim 11, wherein the administration of the said pharmaceutical composition of any of the claims 1 to 8, is administered orally in the form of liquid formulations and preferably as suspension, solution or syrup.
